# EUROPEAN PATENT SPECIFICATION

(11) **EP 3 517 063 B1**
(45) Date of publication and mention of the grant of the patent: **30.03.2022**
(21) Application number: 19153807.3
(22) Date of filing: 25.01.2019
(51) Int. Cl.: A61B 17/70

(54) **COMPRESSION/DISTRACTION DEVICE FOR USE IN SPINAL SURGERY**
KOMPRESSIONS-/DISTRAKTIONSVORRICHTUNG ZUR VERWENDUNG FÜR DIE WIRBELSÄULENCHIRURGIE
DISPOSITIF DE COMPRESSION/DISTRACTION POUR UTILISATION EN CHIRURGIE VERTÉBRALE

(30) Priority: 29.01.2018 US 201815881821
(43) Date of publication of application: 31.07.2019
(73) Proprietor: Globus Medical, Inc., Audubon, PA 19403 (US)
(72) Inventor: BECHTEL, Matthew, PHILADELPHIA, Pennsylvania 19147 (US); JENKINS, Zachary, DOUGLASSVILLE, Pennsylvania 19518 (US)
(74) Representative: Morabito, Sara

(56) References cited:
- EP-A1- 2 692 304
- US-A1- 2006 149 278
- US-A1- 2008 077 155
- US-A1- 2012 239 096
- US-A1- 2013 289 633

## Description

### FIELD OF THE INVENTION

The present application is generally directed to instruments used in spinal surgery and in particular to a compressor/distractor instrument.

### BACKGROUND

Spinal fusion surgery is a common procedure performed to relieve the pain and pressure on the spinal cord that may result from a number of different factors. Generally, spinal fusion may be performed to decompress and stabilize the spine. Spinal fusion generally may entail fusing adjacent vertebrae joints together. The most common drive to perform the surgery may be if a patient experiences degenerative disc disease to the point where the disc wears down and the joint between the vertebrae rubs. Generally, to fuse the adjacent vertebral bodies, the intervertebral disc may be first partially or fully removed. Bone graft may then then typically inserted between neighboring vertebrae to maintain normal disc spacing and restore spinal stability while facilitating an intervertebral fusion.

There are a number of known conventional fusion devices and methodologies in the art for accomplishing spinal fusion. These may include screw and rod arrangements, solid bone implants, and fusion devices which include a cage or other implant mechanism which, typically, may be packed with bone and/or bone growth inducing substances. These devices may be implanted between adjacent vertebral bodies in order to fuse the vertebral bodies together, alleviating the associated pain.

However, there are drawbacks associated with the known conventional fusion devices and methodologies. For example, present methods for adjusting the pedicle screws once drilled within the vertebral bone often may be difficult to accurately tighten or expand. For instance, traditional methods may utilize stabilizer cuffs and a fulcrum, wherein an operator (typically a surgeon) squeezes the stabilizer cuffs at the fulcrum to obtain a desired distance for the screws. This can make it difficult for a surgeon to set the screws a correct distance apart from each other to allow proper fusion.

US2012/239096, US2006/149278

US2013/289633 and EP2692304 describe compression devices known in the art.

### SUMMARY

The invention is defined in independent claim 1. Further advantageous versions of the invention are set forth in the dependent claims.

The present invention provides a compression device for use in a spinal surgery. The compression device comprises a first sleeve having a proximal end and a distal end, wherein the distal end of the first sleeve is arranged for placement over at least a portion of one or more pedicle screw tulip. The compression device further comprises a second sleeve having a proximal end and a distal end, wherein the distal end of the second sleeve is arranged for placement over at least a portion of one or more additional pedicle screw tulip. The compression device further comprises a linear drive, wherein the linear drive is operable to cause movement of the first sleeve and/or the second sleeve for compression and/or distraction.

It is described a method of using a compression device in spinal surgery not falling within the scope of the invention. The method may comprise providing a compression device comprising a first sleeve, a second sleeve, and a linear drive. The method may further comprise disposing a distal end of the first sleeve at least partially over a first tulip of a first pedicle screw in a human body. The method may further comprise disposing a distal end the second sleeve at least partially over a second tulip of a second pedicle screw in a human body. The method may further comprise actuating the linear drive to displace the first sleeve and the second sleeve and, in turn, displace the first tulip and the second tulip for compression and/or distraction.

### BRIEF DESCRIPTION OF THE DRAWINGS

These drawings illustrate certain aspects of some examples of the present invention, and should not be used to limit or define the invention, wherein:
FIG. 1 illustrates an embodiment of a compression device attached to a spinal cord;
FIG. 2 illustrates an embodiment of a compression device;
FIG. 3 illustrates an exploded view of an embodiment of a compression device;
FIG. 4 illustrates an embodiment of a compression device;
FIG. 5 illustrates an exploded view of an embodiment of a compression device;
FIG. 6 illustrates an embodiment of a compression device;
FIG. 7 illustrates an exploded view of an embodiment of a compression device;
FIG. 8 illustrates an exploded view of an embodiment of a compression device;
FIG. 9 illustrates an embodiment of a compression device; and
FIG. 10 illustrates an exploded view of an embodiment of a compression device.

### DETAILED DESCRIPTION

According to the invention it is provided a compression device having the features of claim 1, further devices not falling in the scope of the invention are herein disclosed as examples of compression devices.

Embodiments are directed to spinal treatments and, more particularly, to a compression device for use in spinal surgery that includes integrated compression and distraction. Embodiments of the compression device may include operation in an angulating fashion to enable both compression and distraction with the same device. Additional embodiments may include operation in a linear fashion to enable compression and distraction with the same device. The compression and distraction is performed on pedicle screws disposed in vertebrae. In disclosed embodiments, a linear drive may actuate the compression device to move in an angular or linear fashion to apply force to the pedicle screws for compression/distraction of the vertebrae.

Embodiments of the compression device with integrated compression and distraction may be used in a wide variety of spinal treatments, including spinal fusion surgery. Spinal fusion surgery may be employed to eliminate pain experienced in the spinal cord. One aspect of spinal fusion surgery may be the alignment of at least two adjacent vertebrae prior to fusing them together. Alignment may be done through the use of pedicle screws. In embodiments, a patient may need spinal fusion surgery to fuse two adjacent vertebrae together. A surgeon may first prepare and clean out the area to be treated. The surgeon may then drill the pedicle screws into the two adjacent vertebrae. There may be two pedicles per vertebrae, each requiring a pedicle screw. In embodiments, the pedicle screws may include tulips. The tulips may be disposed about the heads of the pedicle screws. In embodiments, the tulips may be cylindrical and hollow. In embodiments, the inside of the tulips may be threaded. There may be a section of material machined out from opposing sides of the tulip. Once the pedicle screws are drilled, a rod may be disposed within the tulip where material had been machined out. An end of the rod may be disposed in a tulip of a pedicle screw. An opposing end (or portion) of the rod may be disposed in the tulip of the pedicle screw in the adjacent vertebrae. In embodiments, both pedicle screws with the tulips containing the rod may be on the same side of the central axis running along the spinal cord. A set screw may be used to secure the rod within the tulips. In embodiments, the process may be repeated for securely disposing a second rod in the tulips of the remaining pedicle screws. In embodiments, other instrumentation may be used to stabilize the position of the pedicle screws and rods. Compression devices may be used to compress the tulips together in order to obtain a desired distance between them while distraction devices may be used to distract the tulips if the tulips need to be spaced further apart. In accordance with present embodiments, compression and distraction may be integrated into the same compression device.

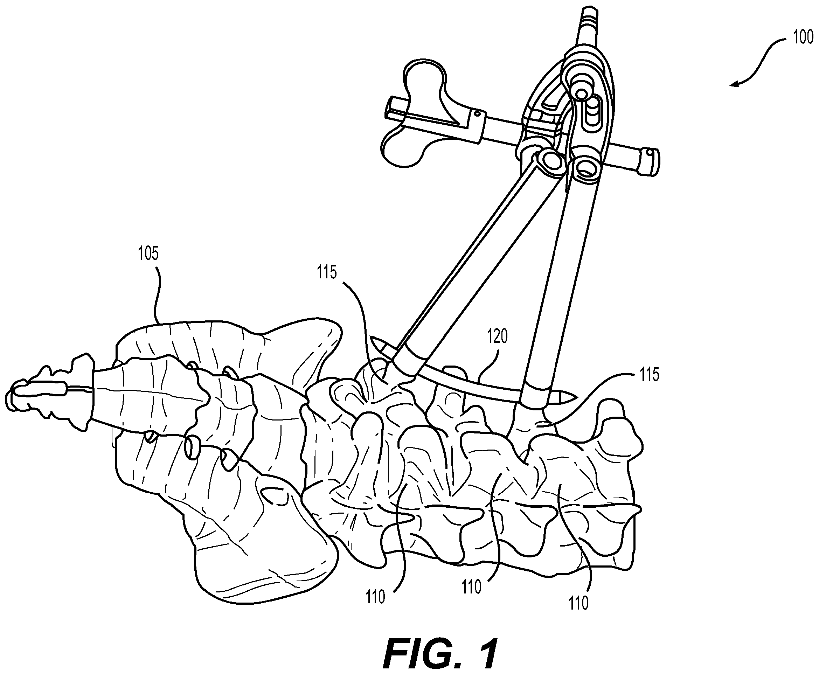

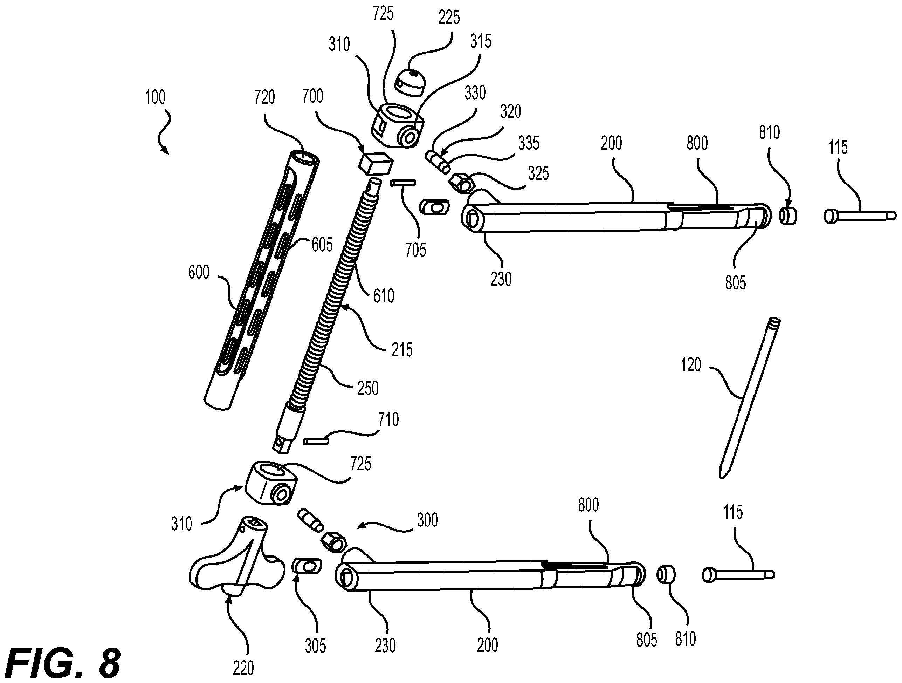

FIG. 1 illustrates an embodiment of a compression device 100 attached to a spinal cord 105, wherein compression device 100 includes integrated compression and distraction. Spinal cord 105 may comprise of a plurality of vertebrae 110. In embodiments, certain vertebrae 110 may be misaligned and/or cause a patient pain. Pedicle screws 115 may be disposed in vertebra 110. Rod 120 may be disposed between pedicle screws 115. Compression device 100 may be utilized in a surgical operation to adjust spinal cord 105. During surgery, compression device 100 may be disposed about the tulips (800 on FIG. 8) of pedicle screws 115 after they have been drilled into vertebrae 110 to be fused. Compression device 100 may exert a force on the tulips of pedicle screws 115 in order to align them a specified distance. By way of example, compression device 100 may exert a compressing force on pedicle screws 115 and corresponding vertebrae 110 close together. By way of further example, compression device 100 may exert a distraction force to increase spacing between pedicle screws 115 and corresponding vertebrae 110.

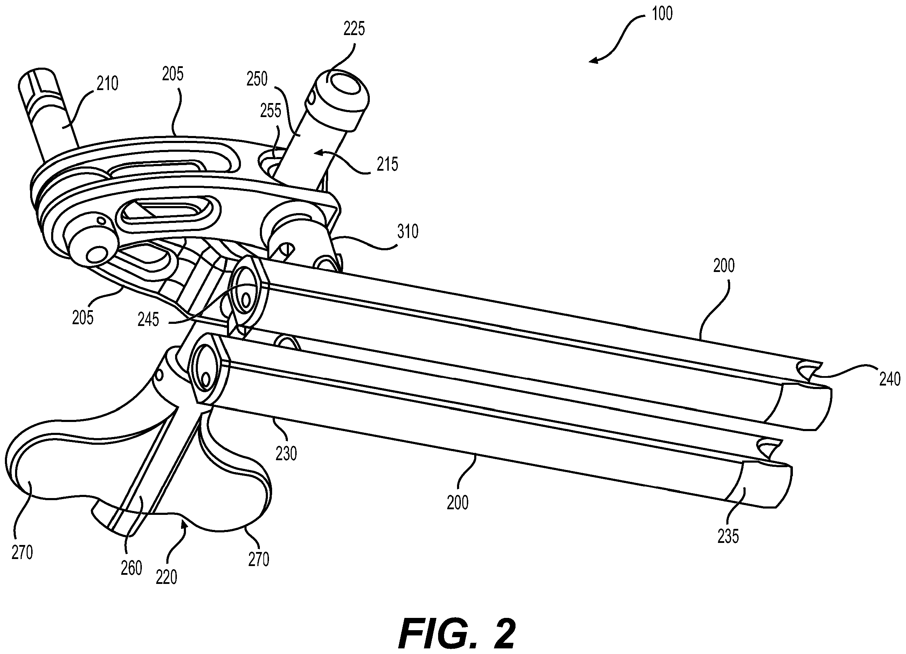

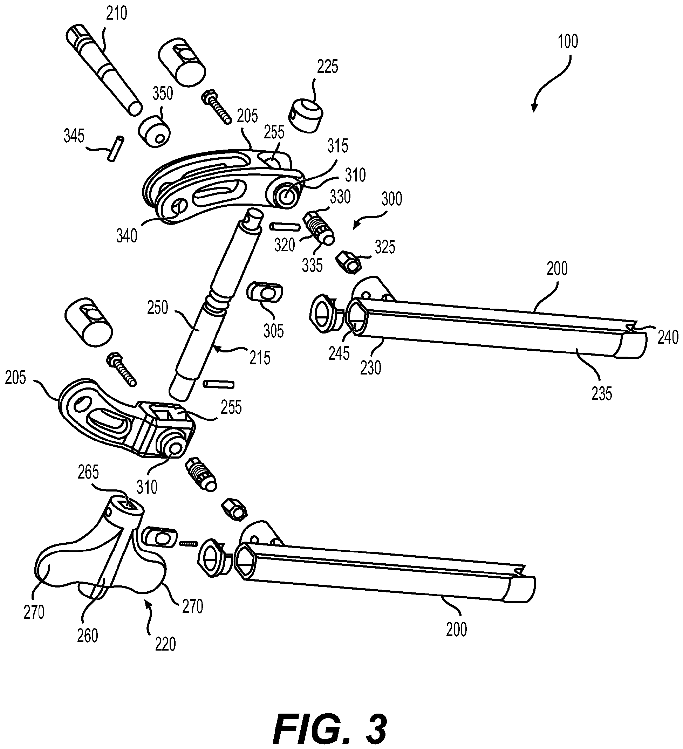

FIGS. 2 and 3 illustrate an embodiment of compression device 100. Compression device 100 comprise of sleeves 200, angulating arms 205, a fulcrum 210, and a linear drive 215. In this embodiment, compression device 100 actuates in an angular fashion in response to linear drive 215. Compression device 100 may be disposed about the tulips of the pedicle screws (e.g., pedicle screws 115 on FIG. 1) in a closed position, partially actuated position, and/or fully actuated position. In embodiments, the closed position may refer to a position wherein sleeves 200 are parallel to each other. In embodiments, a partially actuated position may refer to a position wherein linear drive 215 has moved sleeves 200 to where an angle has formed between them. In embodiments, the fully actuated position may refer to a position wherein linear drive 215 can no longer increase the angle between sleeves 200 (the angle is at its maximum value).

Sleeves 200 may be inserted into a patient's body and attach to the tulips of the pedicle screws (e.g., pedicle screws 115 on FIG. 1). In embodiments, there are two sleeves 200. Without limitation, there may be a plurality of sleeves 200. Sleeves 200 may be made of any suitable material. Without limitation, suitable material may be metals, nonmetals, polymers, composites, ceramics, and/or combinations thereof. In embodiments, sleeves 200 may be made of materials suitable to be safely disposed in a human body. Sleeves 200 may be any suitable size, height, and/or shape. In embodiments, sleeves 200 may be hollow, elongated tubulars. Sleeves 200 may be removable from compression device 100. Sleeves 200 each includes a proximal end 230 and a distal end 235. Proximal end 230 may be secured to angulating arms 205 and distal end may be disposed over pedicle screws (e.g., pedicle screws 115 on FIG. 1).

With specific reference to FIG. 3, compression device 100 further includes spline engagement mechanisms 300, engagement buttons 305, and angulating arms 205 used to attach to sleeves 200. In embodiments, sleeves 200 may attach to angulating arms 205 wherein each attachment point of spline engagement mechanisms 300 may be disposed at an end of a respective one of angulating arms 205. The holes 315 may be used to receive spline engagement mechanism 300. Spline engagement mechanism 300 may comprise of two attachment pieces, illustrated as first attachment piece 320 and second attachment piece 325. First attachment piece 320 may have an end 335 that may fit into an opening in the second attachment piece 325 of spline engagement mechanism 300.

In embodiments, there are an equivalent number of bases 310 as sleeves 200. In embodiments, there may be one or more holes 315 in bases 310. The holes 315 may be threaded. The holes 315 may be used to receive spline engagement mechanism 300. In embodiments, engagement button 305 may comprise a spring and button disposed in proximal ends 230 of sleeves 200. In embodiments, sleeves 200 may lock into place in relation to compression device 100 by pushing engagement button 305 (i.e., applying a force). Prior to locking sleeves 200 in place, an operator may adjust the angle of attachment with sleeves 200 to base 310, and subsequently to angulating arms 205. Spline engagement mechanism 300 may act as a multi-axial joint between angulating arms 205 and sleeves 200. Pushing engagement button 305 may lock sleeves 200 to angulating arms 205 at the desired angle.

Referring to FIGS. 2 and 3, distal ends 235 of sleeves 200 are disposed about the tulips of the pedicle screws (e.g., pedicle screws 115 on FIG. 1). Distal ends 235 of sleeves 200 may have side openings 240 to accommodate the rods (e.g., rod 120 on FIG. 1) connecting the pedicle screws together. Both ends of sleeves 200 may have openings 245 that run the length of sleeves 200. In embodiments, there may be a locking mechanism (not illustrated) to lock sleeves 200 to the pedicle screws. In other embodiments, a driver (not illustrated) may be inserted into sleeves 200, travel along the length of sleeves 200, and tighten and/or loosen the set screws in the pedicle screws.

Sleeves 200 may be operable to move in relation to angulating arms 205. Bases 310 may be secured at an end of angulating arms 205. Once connected to bases 310, proximal ends 230 of sleeves 200 may be secured to angulating arms 205. Angulating arms 205 may transfer motion produced from linear drive 215 to sleeves 200. Without limitation, there may be a plurality of angulating arms 205. Angulating arms 205 may be made of any suitable material. Without limitation, suitable material may be metals, nonmetals, polymers, composites, ceramics, and/or combinations thereof. Angulating arms 205 may be any suitable size, height, and/or shape. In embodiments, angulating arms 205 may be curved. In embodiments, there may be a hole 340 (best seen on FIG. 3) in the end of each of angulating arms 205 opposite of sleeves 200. Hole 340 may be able to accommodate fulcrum 210.

Fulcrum 210 may be a point of rotation for angulating arms 205. Fulcrum 210 may be made of any suitable material. Without limitation, suitable material may be metals, nonmetals, polymers, composites, ceramics, and/or combinations thereof. Fulcrum 210 may be any suitable size, height, and/or shape. In embodiments, fulcrum 210 may be a straight rod. In embodiments, holes 340 in the ends of angulating arms 205 opposite of sleeves 200 may be aligned with each other. Fulcrum 210 may be disposed through holes 340. Suitable fasteners may be used to secure fulcrum 210 within the holes. Without limitation, suitable fasteners may be nuts and bolts, washers, screws, pins, sockets, rods and studs, hinges and/or any combination thereof.

In embodiments, as linear drive 215 is actuated, angulating arms 205 may pivot about fulcrum 210. Linear drive 215 may create motion in a straight line. Linear drive 215 may be made of any suitable material. Without limitation, suitable material may be metals, nonmetals, polymers, composites, ceramics, and/or combinations thereof. Linear drive 215 may be any suitable size, height, and/or shape. Without limitation, linear drive 215 may be any mechanical actuator, hydraulic actuator, pneumatic actuator, piezoelectric actuator, electromagnetic actuator, and/or combinations thereof that produce motion along a single path. In embodiments, linear drive 215 may include a threaded rod 250. Threaded rod 250 may be partially or fully threaded along its length.

In embodiments, angulating arms 205 may be disposed on linear drive 215. As illustrated, angulating arms 205 may be disposed on threaded rod 250. Angulating arms 205 may need to be prevented from sliding off of linear drive 215. A suitable fastener may be disposed about an end of linear drive 215. Without limitation, a suitable fastener may be nuts and bolts, washers, screws, pins, sockets, rods and studs, hinges and/or any combination thereof. In embodiments, a wing nut 220 may be utilized.

Wing nut 220 may prevent an angulating arm 205 from sliding along linear drive 215. In embodiments, wing nut 220 may comprise a body 260, an opening 265, and projections 270 (best seen on Figure 3). Opening 265 may be threaded and may receive linear drive 215. Projections 270 may be portions of body 260 that stick out. Projections 270 may be any suitable size, height, and/or shape that allows an operator to rotate wing nut 220 with a hand. In embodiments, no tools may be needed to rotate wing nut 220. In embodiments, as wing nut 220 is rotated, linear drive 215 may rotate. As linear drive 215 rotates, any equipment attached to linear drive 215 may experience motion. There may be holes 255 disposed through angulating arms 205 about the end wherein sleeves 200 may be attached. Linear drive 215 may be disposed through those holes 255. Holes 255 may be threaded. Holes 255 may be engaged with the threaded rod 250 of linear drive 215. As linear drive 215 rotates, the threaded holes of angulating arms 205 may be displaced as the threaded holes stay engaged with the threaded rod body of linear drive 215. In embodiments, fulcrum 210 may provide a fixed point of rotation. As linear drive 215 is actuated, an angle may form between angulating arms 205 as angulating arms 205 widen. There may be a maximum allowable angle that is defined by manufacturing specifications. In embodiments, linear drive 215 may be actuated in the opposite direction. This may reduce the angle previously formed between angulating arms 205. In embodiments, sleeves 200 may have been attached to angulating arms 205. Sleeves 200 may act as extensions of angulating arms 205. Sleeves 200 may experience the same displacement as angulating arms 205.

In embodiments, a cap 225 may be disposed about the opposing end of linear drive 215 with wing nut 220. Cap 225 may prevent the remaining angulating arm 205 from sliding off of linear drive 215. Cap 225 may be any suitable size, height, and/or shape. In embodiments, cap 225 may be removably attached to an end of linear drive 215. Cap 225 may be affixed to linear drive 215 using any suitable mechanism including, but not limited to, suitable fasteners, threading, adhesives, welding, and/or combinations thereof. In embodiments, a retaining pin may be used to dispose cap 225 about an end of linear drive 215.

There may be a second cap 350 disposed about an end of fulcrum 210. Second cap 350 may prevent fulcrum 210 from sliding out of angulating arms 205. Second cap 350 may be any suitable size, height, and/or shape. In embodiments, second cap 350 may be removably attached to an end of fulcrum 210. Second cap 350 may be affixed to fulcrum 210 using any suitable mechanism including, but not limited to, suitable fasteners, threading, adhesives, welding, and/or combinations thereof. As illustrated, pin 345 may be used to secure second cap 350 to fulcrum 210 while disposed within angulating arms 205.

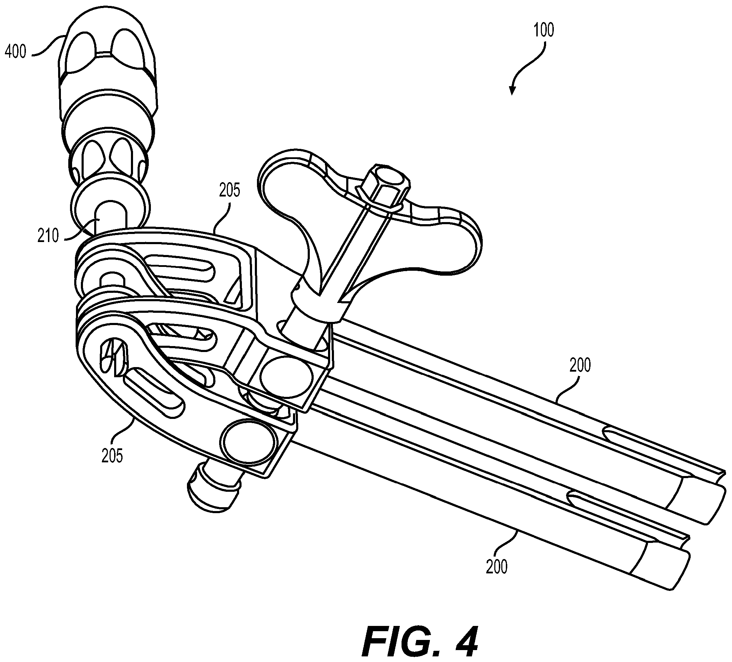

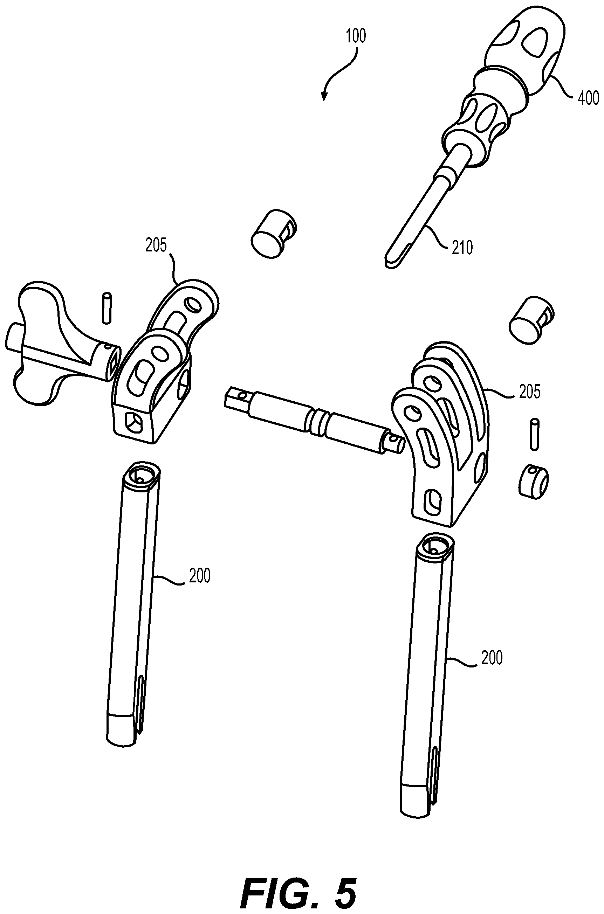

FIGS. 4 and 5 illustrate another embodiment of compression device 100. In this embodiment, compression device 100 may also actuate in an angular fashion. However, sleeves 200 may attach to angulating arms 205 differently than in previous embodiments. Angulating arms 205 may have a hole disposed at an end of each arm. Sleeves 200 may be disposed into each hole. In embodiments, suitable fasteners may be used to secure sleeves 200 within the holes. Without limitation, suitable fasteners may be nuts and bolts, washers, screws, pins, sockets, rods and studs, hinges and/or any combination thereof. There may not be angulation between sleeves 200 and angulating arms 205. There may not be a connecting joint between sleeves 200 and angulating arms 205 (i.e., spline engagement mechanism 300 in FIG. 3). In embodiments, there may be a handle 400. Handle 400 may be disposed about an end of fulcrum 210. Handle 400 may serve as a gripping point for an operator to manipulate compression device 100. Handle 400 may be any suitable size, height, and/or shape.

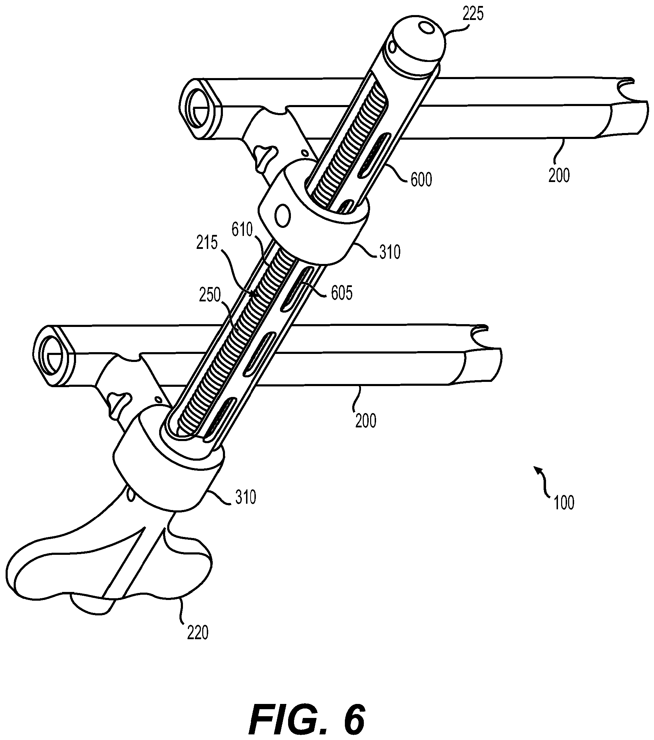

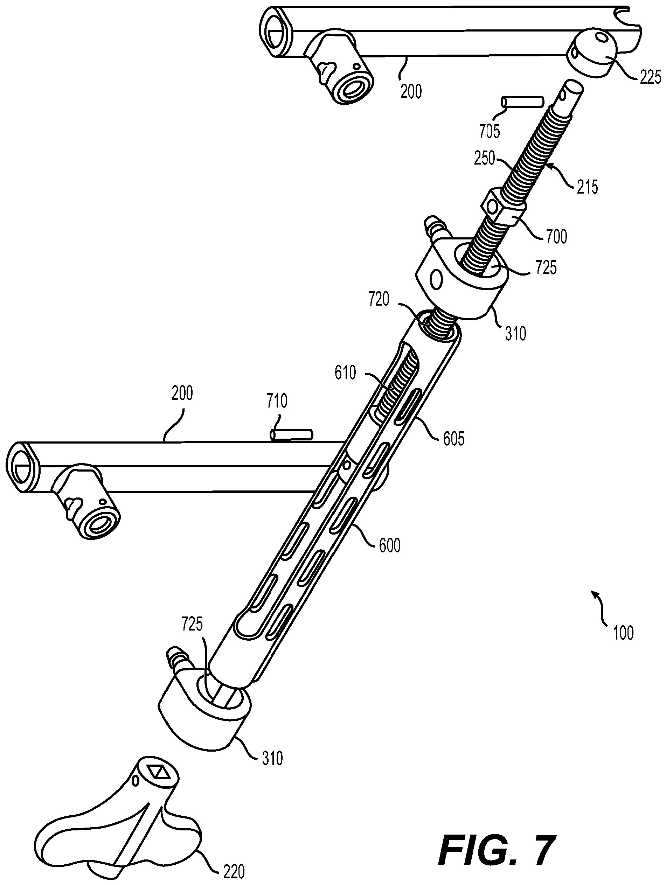

FIGS. 6, 7, and 8 illustrate an embodiment of compression device 100. In embodiments, compression device 100 actuates in a linear fashion. As illustrated, compression device 100 includes sleeves 200, linear drive 215, and sheath 600. Compression device 100 may utilize a sheath 600. Sheath 600 may serve as an outer housing to protect linear drive 215. Sheath 600 may be any suitable size, height, and/or shape. In embodiments, sheath 600 may be an elongated, hollow tubular. Sheath 600 may have holes 605 along its body to permit access and/or visual inspection. Sheath 600 may also have openings 720 at each end to permit the insertion of linear drive 215. Sheath 600 may be disposed around linear drive 215. As illustrated, linear drive 215 may be in the form of a threaded rod 250. Cap 225 may be disposed about an end of sheath 600, for example, to limit the axial movement of linear drive 215 while inside sheath 600. Cap 225 may be any suitable size, height, and/or shape. In embodiments, cap 225 may be removably attached to an end of sheath 600. Cap 225 may be disposed at an end of sheath 600 after the insertion of linear drive 215 into sheath 600. Cap 225 may be affixed to an end of sheath 600 using any suitable mechanism including, but not limited to, suitable fasteners, threading, adhesives, welding, and/or combinations thereof. As best seen on FIG. 7, embodiments may use pin 705 to secure cap 225 to the end of sheath 600.

In embodiments, wing nut 220 is disposed on an opposing end of sheath 600 to cap 225. Wing nut 220 may abut an end of linear drive 215 within sheath 600. In embodiments, retaining pins (e.g., pin 710 shown on FIG. 7) may be used to secure wing nut 220 to a first end of sheath 600 and cap 225 to the opposing end of sheath 600. In these embodiments, bases 310 may not be disposed at an end of angulating arms 205 (referring to FIGS. 2 and 3). In the illustrated embodiments, bases 310 may be disposed around sheath 600. There may be holes 725 disposed through bases 310. Holes 725 may accommodate the shape and size of sheath 600. Sheath 600 may be disposed through holes 725 within bases 310. In embodiments, bases 310 may be fixed and/or moveable. One of bases 310 may be disposed around sheath 600 near the end of sheath 600 where wing nut 220 is disposed. The base 310 nearest wing nut 220 may be fixed in place by using suitable fasteners (e.g., pin 710). The other of bases 310 may be disposed around sheath 600 and further along the length of sheath 600 from wing nut 220 than the other of the bases 310. The one of bases 310 furthest from wing nut 220 may be moveable and may be able to translate axially along sheath 600. In embodiments, either of bases 310 may be moveable with the other of the bases 310 being fixed

A linear moving attachment 700 may engage at least linear drive 215 in at least one of bases 310. Linear moving attachment 700 may be a threaded attachment piece that is engaged with threads 610 of linear drive 215. As linear drive 215 is actuated by wing nut 220, linear moving attachment 700 may translate along a straight path of motion. In the illustrated embodiment, linear moving attachment 700 may be coupled into the one of bases 310 furthest from wing nut 220. Any suitable fasteners may be used to couple linear moving attachment 700 to the corresponding one of bases 310. In embodiments, a retaining pin or retaining post may be used. In embodiments, the corresponding one of bases 310 attached to linear moving attachment 700 may now be forced to move along linear drive 215 as wing nut 220 rotates.

Sleeves 200 attach to bases 310 as previously described in FIGS. 2 and 3. For example, compression device 100 may further include a spline engagement mechanisms 300, engagement buttons 305, and bases 310 used to attach sleeves 200 to bases 310. In embodiments, sleeves 200 may attach to bases 310, wherein each base 310 may be disposed around sheath 600. There may be a plurality of bases 310. In embodiments, there are an equivalent number of bases 310 as sleeves 200. In embodiments, there may be one or more holes 315 in bases 310. The holes 315 may be used to receive spline engagement mechanism 300. The holes 315 may be used to receive spline engagement mechanism 300. Spline engagement mechanism 300 may comprise of two attachment pieces, illustrated as first attachment piece 320 and second attachment piece 325. First attachment piece 320 may have fit into an opening in the second attachment piece 325 using the directed end 335. The second attachment piece 325 may align with engagement button 305. In embodiments, engagement button 305 may comprise a spring and button disposed in proximal ends 230 of sleeves 200. In embodiments, sleeves 200 may lock into place in relation to compression device 100 by pushing engagement button 305 (i.e., applying a force). Prior to locking sleeves 200 in place, an operator may adjust the angle of attachment with sleeves 200 to base 310, and subsequently to sheath 600. Spline engagement mechanism 300 may act as a multi-axial joint between sheath 600 and sleeves 200. Pushing engagement button 305 may lock sleeves 200 to sheath 600 at the desired angle

In previous embodiments, both sleeves 200 may have been actuated to move by rotating wing nut 220. In embodiments, a one of sleeves 200 may be able to move while the other of the sleeves 200 stays stationary. In the illustrated embodiment, the one of sleeves 200 attached to linear drive 215 by way of linear moving attachment 700 may be able to move while the other of the sleeves 200 may be stationary. In this manner, one of the sleeves 200 may move linearly away and/or toward the other of the sleeves 200.

FIG. 8 further illustrates equipment used to secure compression device 100 to specified vertebrae within a patient. Each of sleeves 200 may be disposed over a tulip 800. There may be a plurality of tulips 800. As previously described, tulips 800 may be cylindrical and hollow. In embodiments, the inside of tulips 800 may be threaded. There may be a section of material machined out from opposing sides of each tulip 800. In embodiments, this may form an opening 805. An end of a rod 120 may be disposed within opening 805 of tulip 800. In embodiments, an opposing end of rod 120 may be disposed within opening 805 of an adjacent tulip 800. In embodiments, tulips 800 may be disposed over pedicle screws 115. In embodiments, pedicle screws 115 may be drilled into the vertebrae of the patient. A set screw 810 may be disposed within each tulip 800 to lock tulip 800 around pedicle screw 115. In embodiments, this may also lock rod 120 within tulip 800 to prevent movement of rod 120.

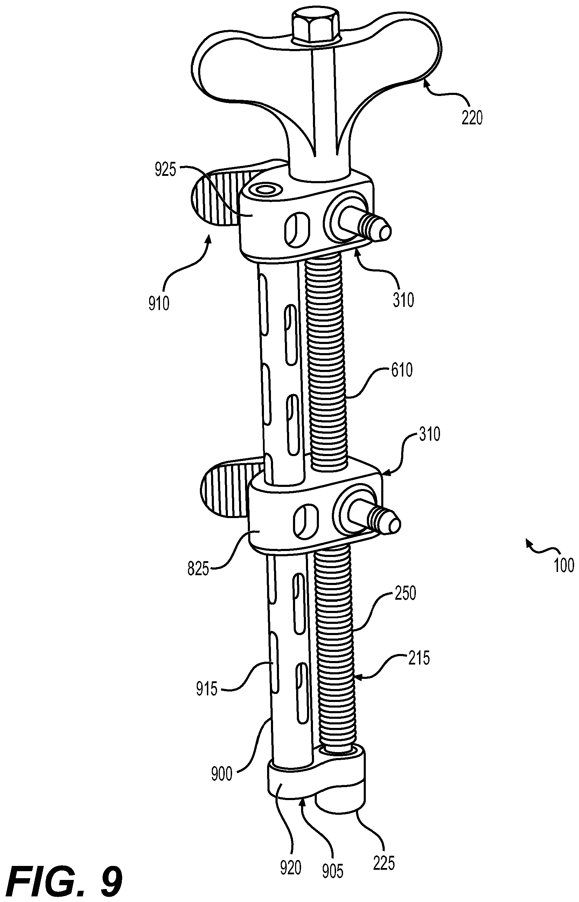

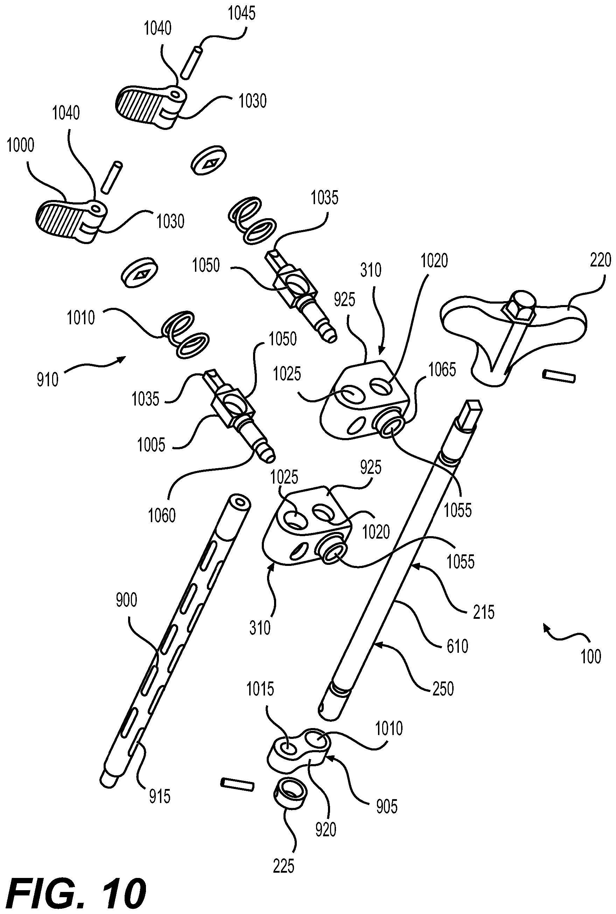

FIGS. 9 and 10 illustrate an embodiment of compression device 100. In embodiments, compression device 100 also actuates in a linear fashion. In embodiments, linear drive 215 may not be disposed within sheath 600 (referring to FIGS. 6, 7, and 8). Linear drive 215 in the form of threaded rod 250 may be exposed to outside elements. Compression device 100 may further comprise of a support bar 900, a connector 905, and a cam lever assembly 910.

Support bar 900 may be used to provide reinforcing support for linear drive 215. Support bar 900 may be made of any suitable material. Without limitation, suitable material may be metals, nonmetals, polymers, composites, ceramics, and/or combinations thereof. Support bar 900 may be any suitable size, height, and/or shape. In embodiments, support bar 900 may be a straight rod. In embodiments, support bar 900 may have holes 915 disposed throughout its body. In embodiments, support bar 900 may be disposed parallel with linear drive 215. Support bar 900 may be adjacent to linear drive 215. Alternatively, there may be distance between support bar 900 and linear drive 215. Support bar 900 may be directly coupled to linear drive through connector 905.

Connector 905 may serve to couple support bar 900 to linear drive 215. Connector 905 may be made of any suitable material. Without limitation, suitable material may be metals, nonmetals, polymers, composites, ceramics, and/or combinations thereof. Connector 905 may be any suitable size, height, and/or shape. In embodiments, connector 905 may have curves and/or fillets. Connector 905 may comprise body portion 920 and two holes 1010, 1015. Linear drive 215 may be disposed through one of the holes 1010. Support bar 900 may be disposed through the other hole 1015. Cap 225 may be disposed about an end of linear actuator. Connector 905 may abut cap 225, wherein cap 225 may prevent connector 905 from sliding off of linear drive 215. Wing nut 220 may be disposed about the opposing end of linear drive 215 (as previously described).

In embodiments, bases 310 are used to attach sleeves 200 (referring to FIGS. 2 and 3) to compression device 100. For simplicity, sleeves 200 are not illustrated on FIGS. 8 and 9. In embodiments, bases 310 may be disposed on both support bar 900 and linear drive 215. Bases 310 may each comprise a body portion 925 and two holes 1020, 1025. Linear drive 215 may be disposed through one of holes 1020. Support bar 900 may be disposed through the other hole 1025.

In embodiments, cam lever assembly 910 may secure linear drive 215 to bases 310. In embodiments, the diameter of holes 1020 of bases 310 wherein linear drive 215 may be disposed may be larger than the diameter of linear drive 215. The diameter of holes 1025 of bases 310 wherein support bar 900 may be disposed may match that of the diameter of support bar 900. Holes 1020 of the bases 310 wherein linear drive 215 may be disposed may be threaded. Cam lever assembly 910 may be actuated to engage the threads of holes 1020 with threads 610 of linear drive 215. Cam lever assembly 910 may be actuated to disengage the threads of holes 1020 with threads 610 of linear drive 215 as well. In embodiments, cam lever assembly 910 may provide translation of bases 310 along linear drive 215 without the need of rotating wing nut 220. Cam lever assembly 910 may be coupled to bases 310 prior to disposing bases 310 on support bar 900 and linear drive 215. Cam lever assembly 910 may comprise of a cam lever 1000, a cam pin 1005, and a cam spring 1010.

Cam lever 1000 may be used as the actuating mechanism within cam lever assembly 910. Cam lever 1000 may be pushed and/or pulled. Cam lever 1000 may be made of any suitable material. Without limitation, suitable material may be metals, nonmetals, polymers, composites, ceramics, and/or combinations thereof. Cam lever 1000 may be any suitable size, height, and/or shape. In embodiments, cam lever 1000 may have a slot 1030 to accommodate cam pin 1005.

Cam pin 1005 may be used to secure bases 310 to linear drive 215. Cam pin 1005 may be made of any suitable material. Without limitation, suitable material may be metals, nonmetals, polymers, composites, ceramics, and/or combinations thereof. Cam pin 1005 may be any suitable size, height, and/or shape. An end of cam pin 1005 may be fitted into slot 1030 of cam lever 1000. There may be a hole 1035 in the end of cam pin 1005, and there may be a hole 1040 running perpendicularly with slot 1030 of cam lever 1000. Hole 1035 and hole 1040 may be lined up. A retaining pin 1045 may be inserted into the holes 1035, 1040 to secure the end of cam pin 1005 into cam lever 1000. There may be a hole 1050 in cam pin 1005 that may accommodate linear drive 215. Cam pin 1005 may be disposed within base 310. There may be a hole 1055 traversing through base 310 where cam pin 1005 may be inserted. In embodiments, hole 1050 in cam pin 1005 that may accommodate linear drive may be concentric with hole 1020 in base 310 wherein linear drive 215 may be disposed through. An end of hole 1055 of base 310 that cam pin 1005 may be inserted in may be ridged. An end of cam pin 1005 may contain a groove 1060. In embodiments, as cam lever assembly 910 actuates, the end of cam pin 1005 that may contain groove 1060 may be forced out of the end of hole 1055 of base 310 that may include a ridge 1065. Ridge 1065 may settle within groove 1060 of cam pin 1005. As this motion occurs, an eccentricity between holes 1050, 1020 may be produced. Hole 1050 in cam pin 1005 that may accommodate linear drive 215 may tangentially align with hole 1020 in base 310 wherein linear drive 215 may be disposed through. This may allow the threads of hole 1020 in base 310 to engage threads 610 of linear drive 215.

In embodiments, cam spring 1010 may provide a compressive and/or expansive force to cam pin 1005 as cam lever 1000 is actuated. Cam spring 1010 may be any suitable spring. Cam spring 1010 may translate the force caused by actuating cam lever 1000 to cam pin 1005, thereby forcing cam pin 1005 to move through base 310. Alternatively, cam spring 1010 may pull cam pin 1005 back, thereby disengaging ridge 1065 from groove 1060 and subsequently the threads of the hole of base 310 with threads 610 of linear drive 215.

In embodiments, sleeves 200 (referring to FIGS. 2 and 3) may be attached to bases 310 once secured to the tulips (e.g., tulips 800 on FIG. 8) of the pedicle screws (e.g., pedicle screws 115 on FIGS. 1 and 8). Cam lever assembly 910 may be actuated to engage the threads of the bases 310 with threads 610 of linear drive 215. Compression device 100 may be actuated to provide a linear displacement between sets of pedicle screws. Once a suitable displacement is obtained, sleeves 200 (referring to FIGS. 2 and 3) may be removed from the tulips of the pedicle screws, and a surgeon may proceed with operation.

## Claims

1. A compression device (100) for use in a spinal surgery, comprising:
- a first sleeve (200) having a proximal end and a distal end, wherein the distal end of the first sleeve is arranged for placement over at least a portion of one or more pedicle screw tulips (800);
- a second sleeve (200) having a proximal end and a distal end, wherein the distal end of the second sleeve (200) is arranged for placement over at least a portion of one or more additional pedicle screw tulips (800); and
- a linear drive (215), wherein the linear drive (215) is operable to cause movement of the first sleeve (200) and/or the second sleeve (200) for compression and/or distraction,
- wherein the linear drive (215) comprises a sheath (600) and a threaded rod (610) at least partially disposed through the sheath (600) and
- wherein the compression device (100) further comprises a first base (310) attached to the proximal end of the first sleeve (200), wherein the sheath (600) is disposed through the first base (310);
- wherein the compression device further comprises a pair of angulating arms (205) coupled to a fulcrum at first end of the angulating arms (205) and to the linear drive (215) at the second end of the angulating arms(205), wherein first end of the angulating arms (205) are rotatable about the fulcrum;
- wherein the second end of one of the angulating arms (205) is coupled to the proximal end of the first sleeve (200) and the second end of another one of the angulating arms (205) is coupled to the proximal end of the second sleeve (200) **characterized in that**;
- the compression device further comprises spline engagement mechanisms (300) coupling each of the angulating arms (205) to the respective one of the first sleeves (200) and the second sleeves (200), wherein the spline engagement mechanisms (300) each comprise a first attachment piece (320) having a threaded end (335) disposed in a
base (310) at the second end of the respective one of the angulating arms (205) and a second attachment piece (325) coupled to the first attachment piece (320) and secured to the proximal end of respective one the first sleeve (200) or the second sleeve (200) with an engagement button (305).

2. The compression device (100) of claim 1, wherein the linear drive (215) comprises a threaded rod (250) and a wing nut (220) at one end of the threaded rod (250).

3. The compression device of claim 1, further comprising a second base (310) attached to the proximal end of the second sleeve (200), wherein the sheath (600) is disposed through the second base (310), wherein the linear drive (215) is operable such that rotation of the threaded rod (250) in a first direction is transferred to the first sleeve (200) and/or the second sleeve (200) as linear motion to cause the first sleeve (200) and the second sleeve (200) to be driven apart while rotation of the threaded rod (250) in a second direction is transferred to the first sleeve (200) and/or the second sleeve (200) as linear motion to cause the first sleeve (200) and the second sleeve (200) to be driven together.

4. The compression device of claim 3, wherein the first sleeve (200) attaches to the first base (310) at an angle, wherein the second sleeve (200) attaches to the second base (310) at an angle.

## Patentansprüche

1. Kompressionsvorrichtung (100) zur Verwendung bei einer Wirbelsäulenoperation, umfassend:
- eine erste Hülse (200) mit einem proximalen Ende und einem distalen Ende, wobei das distale Ende der ersten Hülse zum Anordnen über zumindest einem Abschnitt einer oder mehrerer Tulpen-Pedikelschrauben (800) angeordnet ist;
- eine zweite Hülse (200) mit einem proximalen Ende und einem distalen Ende, wobei das distale Ende der zweiten Hülse (200) zum Anordnen über zumindest einem Teil einer oder mehrerer zusätzlicher Tulpen-Pedikelschrauben (800) angeordnet ist; und
- einen Linearantrieb (215), wobei der Linearantrieb (215) betreibbar ist, um eine Bewegung der ersten Hülse (200) und/oder der zweiten Hülse (200) zur Kompression und/oder Distraktion zu bewirken,
- wobei der Linearantrieb (215) eine Hülle (600) und eine zumindest teilweise durch die Hülle (600) angeordnete Gewindestange (610) aufweist, und
- wobei die Kompressionsvorrichtung (100) ferner eine erste Basis (310) aufweist, die am proximalen Ende der ersten Hülse (200) befestigt ist, wobei die Hülle (600) durch die erste Basis (310) angeordnet ist;
- wobei die Kompressionsvorrichtung ferner ein Paar abgewinkelte Arme (205) aufweist, die mit einem Drehpunkt am ersten Ende der abgewinkelten Arme (205) und mit dem Linearantrieb (215) am zweiten Ende der abgewinkelten Arme (205) verbunden sind, wobei das erste Ende der abgewinkelten Arme (205) um den Drehpunkt drehbar ist;
- wobei das zweite Ende eines der abgewinkelten Arme (205) mit dem proximalen Ende der ersten Hülse (200) verbunden ist und das zweite Ende eines anderen der abgewinkelten Arme (205) mit dem proximalen Ende der zweiten Hülse (200) verbunden ist, **dadurch gekennzeichnet, dass**
- die Kompressionsvorrichtung ferner Keileingriffsmechanismen (300) umfasst, die jeden der abgewinkelten Arme (205) mit der jeweiligen ersten Hülse (200) oder zweiten Hülse (200) verbinden,
- wobei die Keileingriffsmechanismen (300) jeweils ein erstes Befestigungsstück (320) mit einem Gewindeende (335) aufweisen, das in einer Basis (310) am zweiten Ende des jeweiligen einen der abgewinkelten Arme (205) angeordnet ist, und ein zweites Befestigungsstück (325) aufweisen, das mit dem ersten Befestigungsstück (320) verbunden ist und mit einem Eingriffsknopf (305) am proximalen Ende der jeweiligen ersten Hülse (200) oder zweiten Hülse (200) befestigt ist.

2. Kompressionsvorrichtung (100) nach Anspruch 1, wobei der Linearantrieb (215) eine Gewindestange (250) und eine Flügelmutter (220) an einem Ende der Gewindestange (250) aufweist.

3. Kompressionsvorrichtung nach Anspruch 1, die ferner eine zweite Basis (310) aufweist, die am proximalen Ende der zweiten Hülse (200) befestigt ist, wobei die Hülle (600) durch die zweite Basis (310) angeordnet ist, wobei der Linearantrieb (215) derart betreibbar ist, dass eine Drehung der Gewindestange (250) in eine erste Richtung auf die erste Hülse (200) und/oder zweite Hülse (200) als lineare Bewegung übertragen wird, um zu bewirken, dass die erste Hülse (200) und die zweite Hülse (200) auseinander getrieben werden, während eine Drehung der Gewindestange (250) in eine zweite Richtung auf die erste Hülse (200) und/oder zweite Hülse (200) als lineare Bewegung übertragen wird, um zu bewirken, dass die erste Hülse (200) und die zweite Hülse (200) zusammen angetrieben werden.

4. Kompressionsvorrichtung nach Anspruch 3, wobei die erste Hülse (200) an der ersten Basis (310) in einem Winkel befestigt ist, wobei die zweite Hülse (200) an der zweiten Basis (310) in einem Winkel befestigt ist.

## Revendications

1. Dispositif de compression (100) pour son utilisation dans une chirurgie rachidienne, comprenant :
- un premier manchon (200) présentant une extrémité proximale et une extrémité distale, dans lequel l'extrémité distale du premier manchon est agencée pour un placement au-dessus d'au moins une partie d'une ou plusieurs tulipes de vis pédiculaire (800) ;
- un deuxième manchon (200) présentant une extrémité proximale et une extrémité distale, dans lequel l'extrémité distale du deuxième manchon (200) est agencée pour un placement au-dessus d'au moins une partie d'une ou plusieurs tulipes de vis pédiculaire (800) additionnelles ; et
- un entraînement linéaire (215), dans lequel l'entraînement linéaire (215) peut être actionné pour provoquer un mouvement du premier manchon (200) et/ou du deuxième manchon (200) pour une compression et/ou distraction,
- dans lequel l'entraînement linéaire (215) comprend une gaine (600) et une tige filetée (610) disposée au moins en partie à travers la gaine (600) et
- dans lequel le dispositif de compression (100) comprend en outre une première base (310) fixée à l'extrémité proximale du premier manchon (200), dans lequel la gaine (600) est disposée à travers la première base (310) ;
- dans lequel le dispositif de compression comprend en outre une paire de bras en angulation (205) accouplés à un point d'appui au niveau de la première extrémité des bras en angulation (205) et à l'entraînement linéaire (215) au niveau de la deuxième extrémité des bras en angulation (205), dans lequel la première extrémité des bras en angulation (205) peuvent tourner autour du point d'appui ;
- dans lequel la deuxième extrémité d'un des bras en angulation (205) est accouplée à l'extrémité proximale du premier manchon (200) et la deuxième extrémité d'un autre des bras en angulation (205) est accouplée à l'extrémité proximale du deuxième manchon (200) **caractérisé en ce que** :
- le dispositif de compression comprend en outre des mécanismes de mise en prise à cannelure (300) accouplant chacun des bras en angulation (205) à celui respectif des premiers manchons (200) et des deuxièmes manchons (200), dans lequel les mécanismes de mise en prise à cannelure (300) comprennent chacun une première pièce de fixation (320) présentant une extrémité filetée (335) disposée dans une base (310) au niveau de la deuxième extrémité de celui respectif des bras en angulation (205) et une deuxième pièce de fixation (325) accouplée à la première pièce de fixation (320) et attachée à l'extrémité proximale de celui respectif du premier manchon (200) ou du deuxième manchon (200) avec un bouton de mise en prise (305).

2. Dispositif de compression (100) selon la revendication 1, dans lequel l'entraînement linéaire (215) comprend une tige filetée (250) et un écrou à oreilles (220) au niveau d'une extrémité de la tige filetée (250).

3. Dispositif de compression selon la revendication 1, comprenant en outre une deuxième base (310) fixée à l'extrémité proximale du deuxième manchon (200), dans lequel la gaine (600) est disposée à travers la deuxième base (310), dans lequel l'entraînement linéaire (215) peut être actionné de telle sorte qu'une rotation de la tige filetée (250) dans une première direction est transférée au premier manchon (200) et/ou au deuxième manchon (200) en tant que mouvement linéaire pour amener le premier manchon (200) et le deuxième manchon (200) à être entraînés à distance l'un de l'autre pendant qu'une rotation de la tige filetée (250) dans une deuxième direction est transférée au premier manchon (200) et/ou au deuxième manchon (200) en tant que mouvement linéaire pour amener le premier manchon (200) et le deuxième manchon (200) à être entraînés conjointement.

4. Dispositif de compression selon la revendication 3, dans lequel le premier manchon (200) est fixé à la première base (310) selon un angle, dans lequel le deuxième manchon (200) est fixé à la deuxième base (310) selon un angle.
